(19) Europäisches Patentamt
European Patent Office
Office européen des brevets

(11)  **EP 3 527 072 A1**

(12)  **EUROPEAN PATENT APPLICATION**

(43) Date of publication:
  **21.08.2019   Bulletin 2019/34**

(21) Application number: **18156799.1**

(22) Date of filing: **14.02.2018**

(51) Int Cl.:
  **A01M 7/00** (2006.01)   **A01M 9/00** (2006.01)

(84) Designated Contracting States:
  **AL AT BE BG CH CY CZ DE DK EE ES FI FR GB GR HR HU IE IS IT LI LT LU LV MC MK MT NL NO PL PT RO RS SE SI SK SM TR**
  Designated Extension States:
  **BA ME**
  Designated Validation States:
  **MA MD TN**

(71) Applicant: **Agrobio S.L.**
  **04745 El Viso (La Mojonera) - Almeria (ES)**

(72) Inventors:
  • **Pullens, Wilhelmus Martinus Cornelius**
    **5158 NJ Heesbeen (NL)**

• **Vila Rifa, Enrique**
  **04009 Almeria (ES)**

(74) Representative: **Hannke, Christian**
  **Hannke Bittner & Partner**
  **Patent- und Rechtsanwälte mbB**
  **Prüfeninger Straße 1**
  **93049 Regensburg (DE)**

Remarks:
  Amended claims in accordance with Rule 137(2) EPC.

(54)  **METHOD AND DEVICE FOR DISTRIBUTING BENEFICIAL MITES**

(57)  The subject matter of the invention is a method for distributing arthropods in a crop by providing a flow of a liquid medium through a tube in a direction towards a first end of the tube, providing arthropods in a first container, adding arthropods to the liquid medium, allowing the liquid medium to exit the tube at a first end and spraying the mixture of liquid medium and arthropods to a target and a device for spraying arthropods from a first reservoir to a target, wherein the device comprises a second reservoir for a liquid medium which is in fluid connection to a first end of a tube from which the liquid medium exits the tube, wherein the first reservoir comprises a feeding device by which arthropods can be fed to the liquid medium. Furthermore, the invention is directed to a method for protecting a crop.

Fig. 3

**Description**

The present invention relates to a method for distributing arthropods in a crop by providing a flow of a liquid medium through a tube in a direction towards a first end of the tube, providing arthropods in a first container, adding arthropods to the liquid medium, allowing the liquid medium to exit the tube at a first end and spraying the mixture of liquid medium and arthropods to a target.

Furthermore, the invention relates to a device for spraying arthropods from a first reservoir to a target, wherein the device comprises a second reservoir for a liquid medium which is in fluid connection to a first end of a tube from which the liquid medium exits the tube, characterized in that the first reservoir comprises a feeding device by which arthropods can be fed to the liquid medium.

A further aspect of the invention is a method for protecting a crop by providing at least one population of a beneficial arthropod in the cropand providing a food source for the beneficial arthropod species by the method mentioned above or by means of the above-mentioned device.

This method and the device are improving the techniques currently in use when Biological Control programmes are in operation in open and protected crops. In the context of this invention, the programmes relate to the suppression/elimination of pest populations of insects and mites attacking all kinds of crops.

The agents used in Biological Control programmes are themselves insects or mites (Arthropods) which in nature exist by feeding on the same or similar arthropod species known to be the pests. As such, they are termed 'beneficial' agents. A preferred embodiment of the invention is the distribution of beneficial mites, which are one of the most frequently introduced beneficial arthropods onto crops. Most of them are very dependent on high humidity for their survival and development.

Mites are ubiquitous throughout nature, matching insects in numbers of living species, but are much less well known because they are of a size just visible to the naked eye, the largest about 5-6 mm long, the smallest no more than some 60-70 microns. Those used in Biological Control are some of the larger predatory species belonging to the order Mesostigmata, especially from the family Phytoseiidae. The commercial production systems necessary to provide sufficient predator numbers needed in Biological control programmes has been made possible by producing large, dense populations of small, soft bodied mites belonging to the order Astigmata, in particular those belonging to the species collectively known as 'stored product' mites. These mites provide the complete diet of the predators. They are defined as factitious hosts since the two elements predator/prey are almost never found in the same habitats in nature.

Currently, in most developed countries in the world, various combinations of predator and prey populations are being used on a regular basis in crop protection programmes. Some of the most commonly introduced are several species of predatory mites, which are produced and packaged with prey mite populations prior to the distribution into the crops.

Members of the acarine family *Phytoseiidae* have provided the majority of the predators selected as biological control agents for use in protected crops. They are widely used in glasshouses and similar constructions, e.g. polythene structures. Currently, some seven species are being produced on a broad commercial scale worldwide. A summary of the use of these species is given by Griffiths (2003) and van Lenteren (2012).

The current technique most commonly employs a one or two litre cardboard or plastic tube container with a perforated lid. The predators are shaken out through the perforations or through the exit of the tube onto a leaf. All the techniques involving manual dispersing, for instance using these hand-shake bottles, are very labour intensive, with a high economic cost. Moreover, they cannot guarantee an even distribution, especially in dense crops, like chrysanthemums and other cut flowers, where plant density prevents workers from moving within the confines of the crop.

Another method uses blower machines which have been in operation for some time. They are of the same design as garden leaf-blowers, but with a reservoir to hold the predator substrate. An example of these machines is described by Opit et al. (2005). They are much used in plant nurseries, but in general give a very unsatisfactory performance, relative to areas covered, evenness of spread, correct density of predators and accompanying unacceptable predator mortality levels.

A further known system for applying predators to the crop is the "sachet technique", which was invented by Griffiths et al. (see Sampson et al, 1999). The sachet consists of a small paper envelope, into which breeding populations of both the predator and its prey are placed, together with a farinaceous food supply for the prey, plus a small quantity of commercial, bulking substrate composed of bran flakes or vermiculite particles. The envelope is sealed and provided with a small cardboard hook, which is used to hang the sachet on to a plant. A very small hole is pierced in the side of the sachet towards the top margin at the time of filling so that the predators can exit on to the leaf.

The "sachet technique" overcomes, to a considerable extent, the problems caused by discontinuous interruptions in the supply of predators, but again it is an expensive item. The disposition of the sachets in the crop has also a high labour cost, and this system is not plausible for introductions of other arthropods, especially not successful for introduction of prey mites as an in-crop food at the top of the plants on a regular basis.

Since the prey, the astigmatid host, is the food for the predator, both predator and prey can breed whilst inside the sachet. The host usually belongs to a group of mites which are known as "food storage" species and are usually members of the Astigmata. As such they are conditioned to remain inside the sachet, since historically they are used to living in the dark, or in low light levels. Thus, this host tends to stay inside the sachet along with its food supply and, in general, none or just very few will emerge from the sachet, by accident.

Since the host is a food source for the predators, these predators are capable of living inside the sachet for some weeks. They will be well fed when they leave the sachet but if the pest population is not present when they emerge from the sachet, then in a short time they will starve with very few or no survivors if the pests remain absent. If this situation continues over a period of a few weeks, the emerging predators will continue to die so that when the pests eventually arrive, they do so into a virtually unprotected crop, where the pest will quickly become established, forming a stable breeding population in safety.

Many attempts have been made to solve the problem. A solution has recently been found by the inventors of the present invention, in which a further food source for the predatory mite species already in the crop, but outside the sachets, is especially directed to the tops of the plants. This technique has been introduced onto the market where it has been found to be very successful. However, this technique requires the further food source for the predatory mite species to be provided in the crop. Until now this was performed by hand. However, especially for large crops this is very time consuming. Thus, there is the need for devices and methods by which further food sources for the predatory mite species can be provided in the crop quickly and uniformly.

First attempts have been disclosed in the international patent application WO 2007/136246. This patent application discloses a method and device for distributing beneficial arthropods by means of a gas stream. According to the authors the system is an improvement over previous designs because it eliminates or reduces the movement of the arthropods through a duct, accordingly reducing mortality due to the turbulences of the air movement inside the duct. The device is located and moved in a greenhouse above the crop. While moving, the device blows gas and arthropods towards the sides and the arthropods fall down onto the crop due to gravity. However, this device has a plurality of drawbacks and is not suitable to be used outside of greenhouses. The device has to be located above the crop and the uniform distribution is strongly dependent on the weather conditions. Especially wind impedes uniform and thus efficient distribution of the arthropods since at least some of them are blown away. During windy weather conditions sufficient amounts of arthropods cannot be guaranteed at least for parts of the crop. Thus, many problems have not been solved and the major problem with the use of these "air blowing machines" in practice is that it is difficult to maintain the quality of arthropods throughout the application together with the fact that an optimal distribution in the crop is still a big challenge, especially when the area to be treated covers many hectares.

More recently, the Dutch company Pullens M.T.B.V. has designed a new distributing system called "BioSpreader", brought into commercial usage in 2017. Instead of using an air current it relies on a purely mechanical technique namely, having a centrifugal spreader wheel, specifically designed to spread live populations. Trials show the machine can distribute higher densities of live arthropods efficiently and with much less mortality. This spreader can be hung at a stationary position above the crop, but preferably it moves in a longitudinal direction above and along the crop. It can also be mounted to other devices capable of moving alongside the crop. However, this system is limited to flat-bed crops but not for use, for example, in orchards or in low ceiling plastic houses with simple structures, housing vegetables and ornamental crops in Southern Europe, or the open-sided protected tunnels used for strawberries all over the world.

Thus, improved methods and devices are needed. The present invention aims to overcome these disadvantages by providing a usable alternative. In particular the invention aims to provide an arthropod friendly distributor which is able to distribute arthropods over distances in excess of one meter, in such a way that killing or damaging the arthropods is minimized. Further, in particular, this invention will at the same time ensure a uniform distribution over the crop, whilst providing extra humidity, aimed at improving arthropod survival.

These problems are solved by the method according claim 1 and the device according claim 9.

A major aspect of the invention is a method for distributing arthropods in a crop by providing a flow of a liquid medium through a tube in a direction towards a first end of the tube, providing arthropods in a first container, adding arthropods to the liquid medium, allowing the liquid medium to exit the tube at a first end and spraying the mixture of liquid medium and arthropods to a target. This method allows for distributing beneficial arthropods together with a stream of liquid medium. The order of the steps of "adding arthropods to the liquid medium" and "allowing the liquid medium to exit the tube at a first end" is changeable. Depending on a group of parameters comprising the flow characteristics, the species of the arthropods, the liquid medium, the nozzle characteristics, the pressure of the liquid medium, the diameter of the tube and others, the suitable order of these steps can be evaluated.

In a preferred embodiment, the forced flow of liquid medium is generated by a liquid displacer. Most of them generate a pressure introduced by an engine into a container filled with liquid medium, giving force to the flow of liquid medium emerging from the exit hole.

In another preferred embodiment of the invention, a flow of liquid medium is generated by a liquid displacer, which is conducted to an exit. Preferably, the material containing the arthropods expelled from the container is released above or into the flow of liquid immediately after exiting the end of the duct or tube, e.g. through a nozzle. Thus, the arthropods are released outside the duct or tube or similar device that is conducting the flow of liquid medium generated by the liquid displacer. Alternatively, the arthropods can be released inside the duct but very close to the exit, immediately before the liquid flow emerges from the tube or duct. This is important to avoid damaging the arthropods due to the turbulences which may occur inside any duct. "Immediately before the liquid flow emerges from the tube" means that the distance from the point at which the arthropods are added to the liquid medium is less than 20 cm, preferably less than 10 cm, more preferably less than 5 cm and most preferably less than 2 cm with respect to the end of the tube or a nozzle. In the following "end of the tube", "exit of a tube" and "nozzle" are used synonymously, if not specified otherwise.

In a preferred embodiment the liquid medium flow emerges through a nozzle, prior to receiving the arthropods. Modifying the size of the nozzle exit preferably allows for changes of the pressure initially generated by the liquid displacer, which in turn can result in the production of droplets of different size. Adding the arthropods downstream of the nozzle has the advantage, that the arthropods are not subjected to the high pressure changes and accelerations which occur inside the nozzle. However, if the conditions inside the nozzle (or at the exit of a tube) are acceptable for the arthropods, it is possible (and for some arthropods even preferred) to add the arthropods to the liquid medium upstream the nozzle (or at the exit of a tube). The alternative embodiment, in which the liquid medium flow emerges through a nozzle, prior to receiving the arthropods has the advantage that to the jet of liquid medium much more arthropods per litre could be added. Thus, the volume of liquid medium which is needed to apply a defined amount of arthropods to the plants is reduced. This is advantageous since less liquid medium has to be provided in the container, the plants are not treated with a large access of liquid medium and the probability of washing arthropods down from the plants, is reduced.

Preferably, the liquid medium contains water, more preferably the main component of the liquid medium is water. Since the density of the mixture of the liquid medium and arthropods is quite high, the mixture can be distributed over a very wide range and the influence of weather condition, especially wind, is reduced. Preferably, the liquid media is beneficial for the crop or contains further substances which are beneficial for the crop. Thus, more than only one single beneficial substance can be simultaneously applied to the crop.

Furthermore, this method allows application of the mixture of the liquid medium and arthropods from a quite low position. It is not required that the arthropods are applied from a position above the individual plants of the crop. The mixture of the liquid medium and arthropods can be sprayed by means of a spray device from a container located on a vehicle or transported by a worker. Together with the liquid medium, the arthropods could be accelerated upwards and falling down onto plants even if further and/or higher plants are located between the spray device and the target plant/crop. This method allows the provision of the necessary amount of beneficial arthropods to land on a crop in a pre-determined quantity relative to the type of crop, as decided by a worker.

In a preferred variation of the method, the liquid medium is provided from a reservoir, in which it is maintained at a pressure between 10 kPa and 1000 kPa above the atmospheric pressure. In the following all pressures should be understood to mean the difference to the atmospheric pressure. In some cases, the unit "bar" is used for the pressure. In accordance with the International System of Units, 1 bar is defined to be equal to 100000 Pa. Amending the pressure in the reservoir within this range provides one possibility to adapt the characteristics of the spray jet to specific needs, especially the required distance over which the jet can transport the arthropods.

Previous efforts have always concentrated on the use of an air flow to move the arthropod populations because it seemed to be the most "arthropod friendly" system to avoid high mortalities. Results obtained by the applicant have shown that, contrary to the previous understandings, the use of a liquid flow can guarantee a gentle distribution without damaging the populations. Moreover, by using this liquid flow, the material can be dispersed across greater areas whilst at the same time providing added humidity.

In a more preferred variation of the method, the liquid medium is provided from a reservoir, in which it is maintained at a pressure between 100 and 500 kPa, even more preferably between 200 and 400 kPa. These pressure ranges have been found to be especially suitable for the present invention since the spray jet can be extended to the required length and the mortality of the arthropods remains at an acceptable low level. Since the acceleration of the arthropods when entering the jet of liquid medium could be very high at higher pressure differences, higher pressure differences should be avoided or other precautionary measures should be taken to avoid damage of the arthropods.

In a preferred embodiment of the method, the length and/or width of the spray jet is adapted to specific needs by adjusting parameters selected from the group comprising the pressure of the liquid medium in the reservoir, the flow velocity of the liquid medium, the diameter of a section of a tube connecting a reservoir for the liquid medium and the nozzle, the density of the liquid medium, the viscosity of the liquid medium, the cross sectional area of the nozzle and other characteristics of the nozzle. Amending one or more features of this group has been found to be especially suitable to adapt the form of the spray jet to specific needs. Preferably, one or more characteristics of the spray jet selected from the group comprising length, width, height, density, droplet size, opening angle, arthropods per liter of liquid medium and others could be amended. Some of these possibilities (e.g. amending the diameter of a section of a tube or setting the pressure to be zero) will enable the spray jet to be stopped. Stopping the spray jet can be advantageous even during application of arthropods to a crop, e.g. during turns of the vehicle from which the arthropods are sprayed.

As mentioned above, it is desired to maintain a population of the arthropods in the crop to allow the beneficial predators to feed on them. However, sometimes there is not enough food available for the arthropods in the crop to establish a stable population. Thus, in a preferred embodiment a food source for the arthropods is added to and/or together with the liquid medium. Ideally, this food source will be sufficient to allow the arthropods to establish a stable population for over 2 days, preferably more than 4 days, more preferably more than 6 days, more preferably more than one week, even more preferably for more than 10 days and most preferably for more than 2 weeks. Providing a food source for the arthropods which allows maintaining a stable population over such a long time will allow the beneficial predators to be present in the crop for at least the same time. During this period potential pests are arriving into a crop which is already protected by the beneficial predators. Maintaining the arthropods and/or the beneficial predators over a long time will reduce the labor, since sachets and/or arthropods can be applied at longer time intervals.

Preferably, a first reservoir (also called "container") is filled with the at least one pure population of arthropods.

In a preferred embodiment of the method the arthropods are provided together with a carrier to the liquid medium. Such a carrier is often used to rear the arthropods. The carrier material can be any substrate that is suitable to support and disseminate the beneficial arthropods. The technique of providing the arthropods together with a carrier to the liquid medium will reduce the labor because separation of arthropods and carrier can be omitted. Furthermore, it has been shown that arthropods hide in cavities of the carrier, which make them less vulnerable during the application. Especially crucial is the step of adding arthropods to the fluid medium downstream of the nozzle, because of quite high pressure differences and accelerations. Arthropods which are located in cavities of the carrier or which are protected by the carrier from instant contact with the fluid media have higher chances to survive this step.

Preferably, the carrier is selected from a group comprising vermiculite, particles of perlite, saw dust, wood dust, bran, buckwheat, shells of seeds and mixtures thereof. These carriers have been found to be especially suitable for the method. They have also shown to protect the arthropods during the adding-step. Preferably, the carrier (or the substrate) serves as a physical support and carries the populations. Optionally, it can contain food. In a preferred embodiment, the carrier material can be entirely composed of food itself. One example for this embodiment is the usage of bran as carrier, which is also a common food for many astigmatid mites.

Preferably, the carrier (also named "substrate") is pre-conditioned to a moisture content known to be suitable for supporting predators, which provides interstitial spaces containing air, for the populations to breathe during production, storage and transport from bio-factories to the growers, and at the same time introduces the correct humidity level required by the mites.

A further advantage of applying a carrier together with the arthropods is that it can help in the even distribution of arthropods to the tops of the plants. The skilled person will understand how many individuals should be present per volume of substrate, and the needed volumes per surface of crop, which may depend on the crop, the season, area of production and stage of growth of the crop, amongst other factors.

In a preferred embodiment the first reservoir (or container) has at least one exit. Preferably it has two exits, which are for example openings, one to fill the container with material and another one to release the material when the arthropods are being distributed over the crop. Preferably, the filler opening is bigger to facilitate the labor of filling and the other one is smaller, and can be opened or closed with a closing means, for example a valve, which can be a manual valve or a valve automatically monitored using electrical (e.g. battery) power.

Preferably, the opening through which the arthropods are expelled provides a metering device, for instance, a metering powered by hand or with an automatic system, for instance a metering powered with a battery. More preferably, the metering device is communicating (directly or via a control unit) with one or more valves which control the flux of the arthropods and/or the fluid media.

In a preferred embodiment a container is used that has a simple, cheap and efficient system to expel the arthropods with the substrate via an exit. The material moves by gravity to the exit but other mechanisms such as a battery powered vibrator can be included to facilitate the movement of the material. In an even more preferred embodiment such a device can be placed in the exit of the container to facilitate the expulsion of the material through the exit. Other more sophisticated systems, for instance, using electronic devices can be installed which will automatically determine and adjust the volume of substrate emerging from the container.

In a preferred embodiment the method includes spraying the mixture of dispersed liquid and arthropods over a maximum horizontal distance of at least 150 cm. It has been shown that it is advantageous to spray the mixture over such a distance since the spray jet provides a suitable width and even distribution of the arthropods. For most of the crops this distance allows application of arthropods to more than just one row of plants. Thus, multiple rows and/or plants can be targeted at the same time. Preferably, the mixture of dispersed liquid and arthropods could be sprayed over a maximum horizontal distance of at least 200 cm, more preferably of at least 300 cm and especially preferably of at least 500 cm. These preferred embodiments provide targeting of even more rows and/or plants at the same time, giving the required protection with a reduction in labour cost

In a preferred embodiment the method includes selecting the arthropods species from one or more species of insects and/or mites. Predator mites are preferably selected from the order Mesostigmata, more preferably selected from the family Phytoseiidae, most preferably from a group comprising *Amblyseius swirskii, Amblyseius andersoni,*

*Amblyseius largoensis, Euseius stipulatus, Euseius scutalis, Euseius gallicus, Euseius ovalis, Euseius finlandicus, Neoseiulus californicus, Neoseiulus cucumeris, Neoseiulus fallacis, Neoseiulus longispinosus, Amblydromalus limonicus, Transeius montdorensis, Phytoseiulus persimilis* and *Phytoseiulus longipes.* Prey mites are preferably selected from the order Astigmata, more preferably selected from a group comprising the genera *Acarus, Carpoglyphus, Tyrolichus, Thyreophagus, Suidasia, Lepidoglyphus* and *Aleuroglyphus,* most preferably from a group comprising *Acarus siro, Carpoglyphus lactis, Tyrolichus casei, Thyreophagus entomophagus, Aleuroglyphus ovatus, Lepidoglyphus destructor* and *Suidasia medanendis.* It has been shown that these arthropods do not cause damage to the crop whilst at the same time are especially suitable as a food source for beneficial predatory mites in the crop (e.g. those predatory mites brought into the crop by the above-mentioned sachet-technique).

A further aspect of the invention is a device for spraying arthropods from a first reservoir to a target, wherein the device comprises a second reservoir for a liquid medium, which is in fluid connection to an end of a tube, wherein the first reservoir comprises a feed device by which arthropods can be fed to the liquid medium. Such a device simplifies application of arthropods to a crop, and at the same time allows fast distribution of the arthropods over a wide area and/or a large number of plants. Since the device sprays a mixture of arthropods and liquid media, the weather conditions such as wind will have less impact on the distribution of the arthropods.

Preferably, the feed device by which the arthropods are added to the jet is located in vicinity position close to the end of a tube. It has been found that when added to the liquid medium at a distance between less than 20 cm upstream the end of a tube and less than 200 cm downstream of the end of a tube the mortality of the arthropods is low. In this range they are not exposed to harsh conditions like high pressure, sudden pressure differences, high accelerations, high temperature changes and others conditions which may increase mortality to an unacceptable level. The terms "end of a tube", "exit of a tube" and "nozzle" are used synonymously. Preferably, the parameters of the nozzle are adjusted to conditions at which they successfully control the above-mentioned harsh conditions to a level acceptable for the arthropods, thus giving low mortality levels.

In a preferred embodiment of the device the feeding device by which arthropods could be fed to the spray jet is located 0.01 - 200 cm downstream of the nozzle. Feeding the arthropods to the spray jet very close to the nozzle allows for a very homogeneous distribution of the arthropods within the spray jet. However, the physical forces to which the arthropods are exposed during the feeding step and which could harm the arthropods will be very high at positions close to the nozzle especially due to the high acceleration and the high density of the liquid media within the spray jet. The acceleration and the density can be reduced at positions more remote from the nozzle by widening the jet and reducing jet-speed (e.g. due to the aerodynamic drag and turbulences). However, if the arthropods are feed to the widened jet (e.g. at its center) only this part of the jet will be mixed with arthropods whilst the other parts (e.g. the outermost parts) will only contain the liquid medium (and the optional further ingredients added upstream with respect to the nozzle). Thus, a location for the feeding step must be found at which the arthropods are not harmed to a large extent and a homogeneous distribution of the arthropods in the jet is achieved. The optimal location depends on the species of the arthropods, the presence of a carrier and the characteristics of the spray jet and the liquid medium. It has been found, that a location of 1 - 50 cm downstream of the nozzle is especially suitable for most conditions. Depending on the characteristics of the spray jet and the liquid medium, for most arthropods a location of 2 - 10 cm downstream of the nozzle has been found the most suitable.

The first reservoir (or container) can be a unit formed from any material as long as it is suitable for holding the beneficial arthropods. A preferred embodiment is any type of plastic transparent material through which the amount of remaining material inside the container can be seen at any time.

In a preferred embodiment the container is made at least in parts from a transparent material, for instance, plastic, that allows the amount of material remaining inside to be seen at any time.

Preferably, the first reservoir (in the following also named "container") is refillable, according to the needed volumes of material to be applied to a specific crop, and the surface desired to be covered. The container preferably holds a volume of beneficial arthropods between 0.1 liters and 300 liters, preferable between 0.5 and 100 liters, rather between 1 and 50 liters, most preferably between 2 and 20 liters. The volumes can vary depending whether the device will be held in the hand, or on the shoulders or is housed in a structure, which can be moved throughout the crop, for instance, a trolley, a pipe or cable, an irrigation system, or a motorized vehicle, among other options.

The flow rate of water spread is between 0.10 liters per minute to 2810 liters per minute, preferably between 0.16 and 725 liters per minute, most preferably between 0.16 and 100 liters per minute, even most preferably between 0.64 and 1,29 liters per minute.

The speed of the water depends on the pressure of the water, and can be varied between 0.5 to 10 meters per second, preferably between 0.78 and 6.63 meters per second, most preferably between 0.78 and 1.58 meters per second.

In a preferred embodiment of the device, the device comprises adjustment means for allowing and/or preventing flow of the liquid medium through the nozzle and/or adapting parameters of the spray jet selected from the group comprising the lengths, width, height, opening angle, diameter, form, velocity, flow rate of the spray jet. These means for allowing and/or preventing flow of the liquid medium through the nozzle and/or adapting parameters of the spray jet have been found to be suitable to adjust the spray jet to specific needs or to start, pause or terminate the spraying procedure. Starting, pausing or terminating the flow of the liquid medium and thus also the spraying procedure may for example be important when using a vehicle which turns outside the crop to re-enter the crop in a new row of plants. Adapting parameters of the spray jet is helpful to adjust the spray jet to specific needs or conditions. By adapting the above-mentioned parameters, e.g. the target area, the amount of liquid and/or arthropods per liter and/or square meter, the distance between the nozzle and the target plant(s) can be optimized.

Preferably, the system has from 1 to 30 nozzles, preferably between 1 and 10, most preferably between 1 and 3. More nozzles allow targeting a larger area at the same time. Thus, the method for applying arthropods can be even more efficient.

Preferably, the size of the nozzle's opening hole is between 0.1 mm and 1.3 mm. These openings allow suitable flow rates for applying arthropods.

Preferably, the pressure of the liquid media in the reservoir is in the range of 0.5 to 30 bar, preferably between 1 and 6 bar, and more preferably between 2 and 3 bar.

The flow rate of water spread is between 0.10 liters per minute to 2810 litres per minute, preferably between 0.16 and 725 liters per minute, most preferably between 0.16 and 100 liters per minute, most preferably between 0.64 and 1,29 liters per minute.

Preferably, the speed of the water depends on the pressure of the water, and can vary between 0.5 to 10 meters per second, preferably between 0.78 and 6.63 meters per second, most preferably between 0.78 and 1.58 meters per second.

The droplet size of the exit fluid after the nozzle will vary according to the nozzle used. But it has to be understood that the liquid can also be sprayed without using a nozzle, then the liquid flows without droplets. A preferred system uses a nozzle which forms a droplet size (with a mean droplet diameter) between 0.04 and 5 mm, more preferably between 0.08 and 0.22 mm, even more preferably between 0.08 and 0.17 mm, in a preferred embodiment of about 0.08 mm. There are also some chemical products, like surfactants, that can be incorporated into the fluid which can decrease the size of the droplets. Products that do not damage the living populations of arthropods have to be chosen, for instance some molten alkali silicates.

Preferably, the angle of spread (opening angle) is between 10° and 180° degrees. More preferably the spreading angle can be adjusted in this range to specific needs. Angles in this range allow targeting single plants (small angles) as well as a plurality of plants (great angles) at the same time, giving improved efficiency.

In a preferred embodiment the device provides a control device which controls the arthropods to be fed to the liquid medium at a ratio between 1 and 200 grams per minute. This ratio has been determined to provide an even and high density of arthropods in the crop, depending on the flow rate of the liquid medium. Values between 3 and 80 grams per minute, preferably between 5 to 40 grams per minute, more preferably between 10 and 30 grams per minute have been found to be especially suitable.

Preferably, the material has a mean number between 1 and 400.000 individuals of the arthropods per gram. It has been shown that this density allows good handling of the arthropods, even in large quantities. The prey mites as food for the predators are preferably released at higher numbers than the predators, in order to supply populations preferably "at libitum". In this case the predators will have plenty of alternative food available on the plants. Thus, the densities of arthropods per gram strongly depends on the kind of arthropods in use. If the arthropods are predatory arthropods (e.g. predatory mites), the mean number of arthropods is preferably between 1 and 3.000 individuals per gram, preferably between 4 and 1600 individuals per gram, more preferably between 40 and 600 individuals per gram, most preferably between 100 and 400 individuals per gram. For arthropods (astigmatid mite species) which should serve as a food source for the beneficial predators, higher densities between 4 and 400.000 individuals per gram have found to be adventitious. Preferably, the density is between 400 and 200.000 individuals per gram, more preferably between 2.000 and 80.000 individuals per gram and most preferably between 4.000 and 40.000 individuals per gram.

Preferably, the device comprises a valve for adjusting the pressure of the liquid medium. The flow rate of the liquid medium, which for example can be adjusted by the pressure (difference), has been found to be a major factor for influencing the parameters (e.g. length and/or width of the jet, droplet size) of the jet. The distance travelled by the arthropod from the end of the nozzle to the crop can be changed by amending the pressure of the liquid medium. Controlling the pressure is also important in order to avoid damaging the living populations of arthropods. Preferably, the pressure of liquid medium in the reservoir (difference to the ambient pressure) is between 0.1 and 10 bares (10000 - 1000000 Pa), preferably between 1 and 5 bares (100000 - 500000 Pa), most preferably between 2 and 4 bares (200000 - 400000 Pa).

Depending on the pressure of the liquid flow, the populations of arthropods will be distributed in different distances, which will also be influenced by gravitational pull and the resistance offered by the air. The invented method has shown that the system can distribute the arthropod populations in a distance of up to 5 meters without damaging the living populations. In fact it is possible to have less than a 10 % mortality of the vulnerable arthropods.

In a preferred embodiment of the device the second reservoir for the liquid medium is portable. This allows application of the mixture of arthropods and liquid medium independently from local availability of the liquid medium. In one example of the device, the second reservoir is a container which can be carried by a worker on his back. This container is connected with the nozzle by a flexible tube. Thus, the worker can direct the spray jet to any target independently of the position of the container. As is described above, it is preferred that the second reservoir is portable together with the nozzle. However, a second reservoir and nozzle could also be spaced apart from each other. For example, it is possible that only the second reservoir is located on a vehicle, whilst the nozzle is transported by a worker, who can target a desired area with the spray jet. However, in an especially preferred embodiment the second reservoir as well as the nozzle are both located on a vehicle. This embodiment allows a worker to drive the vehicle through the crop and directing the spray jet remotely controlled from the drivers cab. Controlling the spray jet remotely from the drivers cab requires means (e.g. servo motors) to direct or adjust the nozzle based on the control signals emitted by a control device located in the drivers cab or any other control stand.

In a preferred embodiment of the device the first reservoir comprises a dosing device by which the number and/or weight of emitted arthropods per minute and/or volume of sprayed liquid medium can be adjusted. It has been shown that not only the amount of liquid medium ejected from the nozzle and/or the shape of the spray jet is crucial for providing a desired density and/or number of arthropods into the crop. Especially if an even distribution of arthropods in the crop is desired, also the number and/or weight of arthropods per liter and/or minute should be adjusted. Thus, it is preferred that the number and/or weight of emitted arthropods can be adjusted to specific needs independently from the volume of sprayed liquid medium or the form of the spray jet.

In a preferred embodiment of the device the arthropods located in the first reservoir are one or more species of insects and mites. Predatory mites are preferably selected from the order Mesostigmata, more preferably selected from the family Phytoseiidae, most preferably from a group comprising *Amblyseius swirskii, Amblyseius andersoni, Amblyseius largoensis, Euseius stipulatus, Euseius scutalis, Euseius gallicus, Euseius ovalis, Euseius finlandicus, Neoseiulus californicus, Neoseiulus cucumeris, Neoseiulus fallacis, Neoseiulus longispinosus, Amblydromalus limonicus, Transeius montdorensis, Phytoseiulus persimilis* and *Phytoseiulus longipes.* Prey mites are selected preferably from the order Astigmata, more preferably selected from the genus *Acarus, Carpoglyphus, Tyrolichus, Thyreophagus, Suidasia, Lepidoglyphus* and *Aleuroglyphus,* most preferably from a group comprising *Acarus siro, Carpoglyphus lactis, Tyrolichus casei, Thyreophagus entomophagus, Aleuroglyphus ovatus, Lepidoglyphus destructor* and *Suidasia medanendis.* It has been shown that members of these types of arthropods are especially suitable for maintaining a stable population of beneficial predatory mites in the crop.

Furthermore, it has been shown to be advantageous that more than one species of arthropods is located in the first reservoir (and thus applied to the crop), since it might be possible that one of these species is not suitable to establish a stable population in the crop due to external influences (e.g. other (unwanted) predators, insecticides, harsh weather conditions). Thus, applying one or more additional species of arthropods to the crop will increase the chances that one of these species will remain in the crop for a longer time to allow the beneficial predators to prey on it, thus establishing a stable population. In a further preferred embodiment, one or more of the prey species is selected from a group comprising *Acarus siro, Carpoglyphus lactis, Tyrolichus casei, Thyreophagus entomophagus, Aleuroglyphus ovatus, Lepidoglyphus destructor* and *Suidasia medanendis.* These species have been found to be especially suitable prey for commonly used beneficial predators. Furthermore, members of these species can survive a wide range of weather conditions over a long period. Thus, application of new arthropods to maintain a stable population of the beneficial predators in the crop until a pest arrives could be avoided in many cases.

In a preferred embodiment of the device the first reservoir further contains a carrier which is selected from a group comprising vermiculite, particles of perlite, sawdust, wood dust, bran, buckwheat, shells of seeds and mixtures thereof. As mentioned above the carrier will serve the arthropods to hide within cavities. This increases the chances that the arthropods survive the accelerations and the change of conditions when added to the spray jet. Thus, the carrier reduces mortality of the arthropods and allows higher densities to be applied to the crop in a single spray procedure.

In a preferred embodiment of the device the device comprises means for adding to the liquid medium a food source for the arthropods. This allows for application of such a food source together with the arthropods and the liquid medium to the crop. Thus, it is guaranteed that a suitable food source for the arthropods is present in the crop at least for some time and the arthropods can use this food source for maintaining a stable population over at least some days. If water is used as liquid medium, the chances are very high that all nutrients for the arthropods are present in the crop. The food source is preferably selected from a group comprising pollen, sugar, amino acids, vitamins, nutrients derived from insect tissue, eggs, preferably mite eggs, moth eggs, crustacean cysts and/or mites, preferably dead mites. These food sources have been shown to be acceptable for most of the applied arthropods, especially those species mentioned above.

A further aspect of the present invention is a method for protecting a crop by providing at least one beneficial arthropod, preferably a rearing population of a predator mite species of the group of Mesostigmata, most preferably of the family Phytoseiidae, in the crop and providing a food source for the beneficial arthropod species according to the method described above and/or by means of a device as described above. This method has been found to be especially suitable since the beneficial arthropod species will be maintained in the crop for a long period (up to weeks or even months (eventually with additional applications of arthropods if necessary)) with very little effort. Since the application of arthropods could be performed by a device as described above (e.g. mounted on a vehicle) and/or the method as described above, a single worker could easily apply sufficient arthropods to many hectares or even square kilometers per day.

In a preferred embodiment of the method for protecting a crop the at least one rearing population of a predator mite species of the family Phytoseiidae is provided together with a first food source for this predator mite species in the crop. More preferably the rearing population of the predator mite species and the first food source are both located in a common housing, which has at least one opening which allows individuals of the predator mite species to leave the common housing. More preferably a second food source for the predator mite species is provided outside the common housing according to the method described above and/or by means of a device. The second food source and the first food source can comprise the same or different arthropods species, preferably mite species.

Preferably the device for distributing the arthropods provides, at the same time as it is distributing the arthropods, a controlled source of liquid which considerably improves the humidity levels within the close vicinity of the plants. This humidity benefits the survivorship and development of the supplied populations of beneficial arthropods.

The dispersal components are not limited to astigmatid prey mites. They may for example include mixed populations of predator and prey as well as pure populations of astigmatid prey mites, optionally together with a filler substrate composed of vermiculite particles or bran flakes, or similar carrier materials. The method of application to the crop can differ relative to a particular crop husbandry and to the size of the operation. Across the world, in the protected crop industry, the structural units enclosing the crops vary from very large state of the art glass and plastic houses to small primitive structures covered in plastic sheeting.

The present invention is especially versatile for use in all of the above-mentioned situations, as well as in open field crops, including for instance, orchards. It is intended to use it to replace current methods of distribution of beneficial arthropods which are inefficient in respect of even distribution, distance of distribution, density cover of predators, and/or the expense in cost and labour requirements of the producer and the end user. In several embodiments of the invention further measures are taken to make the distribution of the beneficial arthropods, for instance the distribution of mites, more effective than the methods known in the state of the art.

The optional provision of extra humidity (in case the liquid medium is or contains water) is especially convenient for the food storage astigmatid mites used specifically as prey for phytoseiid predators, since they are different to all other mite groups in that they breathe through their skin, which makes it difficult to maintain an inner water balance. Which is why the common habitat of these astigmatid species is deep inside stored food parcels and, with very few exceptions, are never naturally found on plant leaves.

In a preferred embodiment the present invention is designed to distribute pure populations of astigmatid mites (together with the liquid medium), as an in-crop food to the top of the plants.

Preferably, the arthropods being expelled through the exit of the container are directed on to a liquid flow, preferably to a water flow, by the action of gravity or a mechanical device.

Trials performed by the applicant have shown that additional survival can be obtained when using the invention compared to previous air blowing distributors, due in considerable part to the added humidity. Another advantage is that the arthropods and carrying substrate attaches more firmly to the surface of the top leaves of the crop, because they stick easier due to the presence of a water film. Again ensuring more predators reach their target.

In a further embodiment of the invention, the method can be used to distribute dead populations of astigmatid prey species which have been previously fast frozen, bringing the option of feeding predatory mites to the top of plants with not only those species commercially available but also a few species which are otherwise unavailable because they represent very few astigmatid species which are actually pests of crops. An example being *Tyrophagus pernicious,* which in recent years has become a worldwide pest infesting spinach crops. Predators in these crops can now be fed dead individuals of their pest diet, which is harmless harmless to the crop. Another example is the introduction of UV or frozen treated eggs of *Ephestia kuheniella,* which are currently distributed mostly by hand, in many greenhouse tomato crops as food for predatory bugs. A practice which is labour intensive. Similarly, sterilized eggs of the Mediterranean fruit fly *Ceratitis capitata,* and cists of *crustaceans,* known as brine shrimp eggs, Artemia, can also be distributed much more economically.

In a further preferred embodiment of the invention certain foliar nutrients and/or bio-stimulants are added and simultaneously applied to the crop. The foliar nutrients and/or bio-stimulants are not toxic to the beneficial arthropods being distributed.

Further advantages, aims and characteristics of the present invention are explained with reference to the appended drawings and the following description in which embodiments of the method and/or the system are illustrated and described by way of example. The figures show:

Fig. 1:         illustration of a device of the present invention according to a first embodiment,

Fig. 2:     detailed illustration of a first embodiment of the feeding device, by which the arthropods are added to the liquid medium,

Fig. 3:     detailed illustration of a second embodiment of the feeding device, by which the arthropods are added to the liquid medium,

Fig. 4:     detailed illustration of a third embodiment of the feeding device, by which the arthropods are added to the liquid medium,

Fig. 5:     illustration of adapted spray jet characteristics (opening angle),

Fig. 6a, 6b:     illustration of spray jet characteristics (flat spray jet), and

Fig. 7a, 7b:     illustration of a further embodiment of the present invention with two nozzles.

Fig. 8:     Mean number ($\pm$EE) of eggs and mobile stages of *T. montdorensis* counted per plant on each sampling date where the mites where introduced using the invention,

Fig. 9:     Mean number ($\pm$EE) of eggs and mobile stages of *T. montdorensis* counted per plant on each sampling date in the treatment where the mites were introduced using the BioSpreader,

Fig. 10:    Mean number ($\pm$EE) of thrips counted per plant on weeks 1, 3 and 4 in the treatment where the mites where introduced using the BioSpreader.

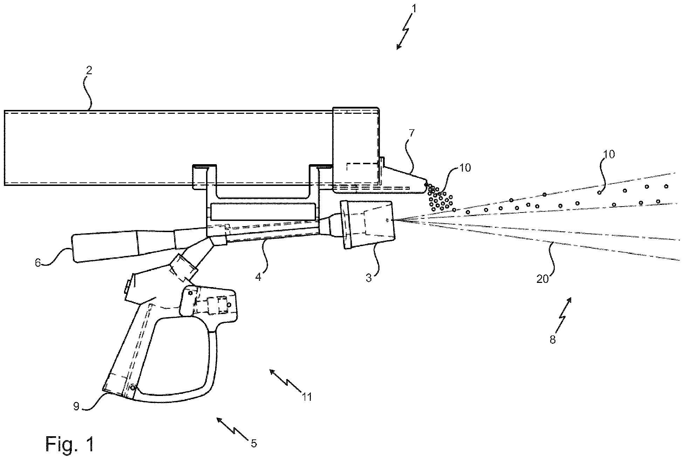

Figure 1 shows an exemplary embodiment of a device 1 for spraying arthropods 10 according to the invention. This device comprises a first reservoir 2 in which the arthropods 10 can be located. This reservoir comprises at least one opening from which arthropods 10 can exit to be fed to the liquid medium 20. Preferably, the first reservoir 2 comprises a further opening for filling the arthropods 10 into the reservoir.

The second reservoir for the liquid medium 20 is not shown. This second reservoir is in fluid connection to an end 3 of a tube 4. The end 3 of a tube 4 can be an adjustable nozzle like it is shown in Figure 1. The device 1 further comprises (in this case two) control units 5 and 6. The first control unit 5 allows on-off control of the flow of the liquid medium. The second control unit 6 allows adjustment of the spray jet characteristics by adapting the nozzle 3 to specific needs.

The first reservoir 2 comprises a feed device 7 by which arthropods 10 could be fed to the liquid media 20. In the illustrated example, the feeding device 7 is located downstream of the nozzle 3. Thus, the arthropods 10 are fed directly to the spray jet 8 formed by the liquid medium 20. The feeding device 7 is a closing system for the first opening. The feeding device 7 could for example be valve, suitable to regulate the volume of the material containing the arthropods that can pass throughout the first opening. In a further embodiment, the feeding device 7 is dosing the arthropods 10 by a mechanical system which transports the material outside the reservoir 2. In a further embodiment of the invention the exit of the container optionally contains a metering device, which can be a simple system, moved by hand or a sophisticated system, for instance, with a battery and/or with an electronic system to control the volume of material to be released by time. In the shown system, the introduction of the arthropods 10 into the jet 8 is done by aid of gravity or by aid of a mechanical system, for instance, by a vibrating device, conveyor belt or rotating screw.

The arthropods are fed to the liquid medium at a ratio between 1 and 200 grams per minute, preferably between 3 and 80 grams per minute, more preferably between 5 and 40 grams per minute, even more preferably between 10 and 30 grams per minute.

The pressurized fluid medium enters the machine through a further opening 9 with a pressure preferably between 0.1 and 30 bar. In the illustrated embodiment a male pipe could be connected to the shown spray gun for water supply. Preferably, the fluid exits through a nozzle system 3, in order to save the amount of water dispensed at the top of the plants.

The device 1 according to the shown embodiment further comprises a system 11 to hold by hand the end of the duct with the exit of the flow of liquid where the arthropods 10 are introduced, so that the flow of liquid containing the arthropods can be directed to the target plants (not shown). The device optionally includes other means to carry the device and to move it through or above the crop.

The device comprises a liquid displacer that includes a container with the liquid and a pump or any other means (not shown) to move the liquid of the container through a duct, thus generating the liquid flow.

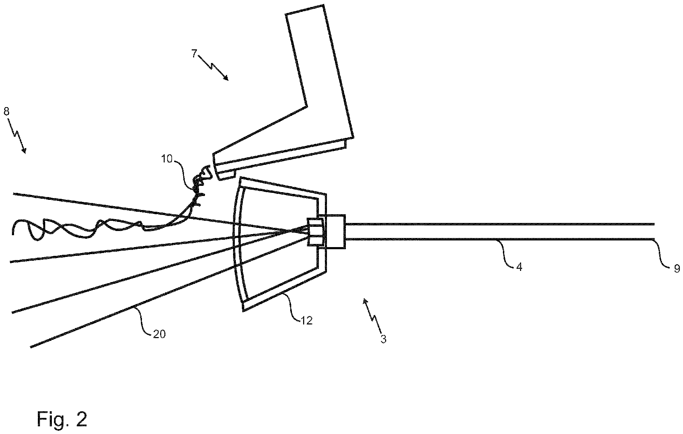

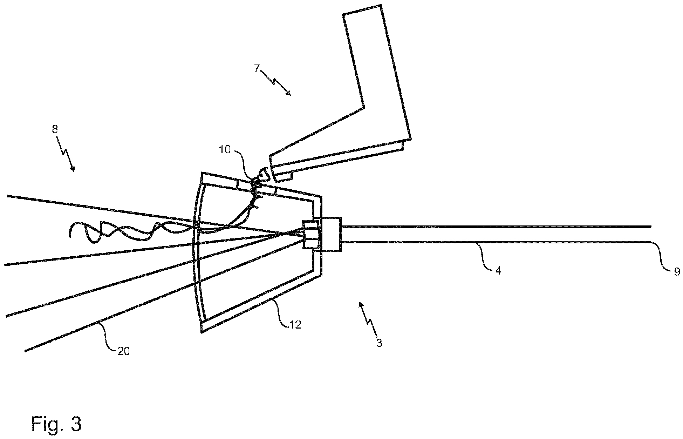

Figures 2 to 4 show several details of preferred embodiments of the invention. They illustrate different embodiments of the system, by which the arthropods 10 are introduced into the liquid medium 20. As shown in Figure 2, arthropods 10 could be fed to the liquid medium 20 downstream with respect to the nozzle 3. In the illustrated example, the nozzle comprises a shield 12. This shield encompasses the nozzle 3 and prevents contaminations of the nozzle 3. Even if the device falls down on earth or in mud, dirt cannot enter into the nozzle 3.

Furthermore, the shield 12 reduces effects of the surrounding conditions (e.g. wind) to have negative effects on the formation of the spray jet 20 and prevents arthropods 10 to enter into the nozzle 3.

It has been found, that in the embodiment shown in Figure 2, strong winds can blow away (small) parts of the arthropods 10 prior they are introduced into the liquid medium 20. Thus, a modified shielding 12 has been designed, by which the distance, at which the arthropods 10 are exposed to wind on its way from the feeding device 7 to the spray jet 8 is reduced.

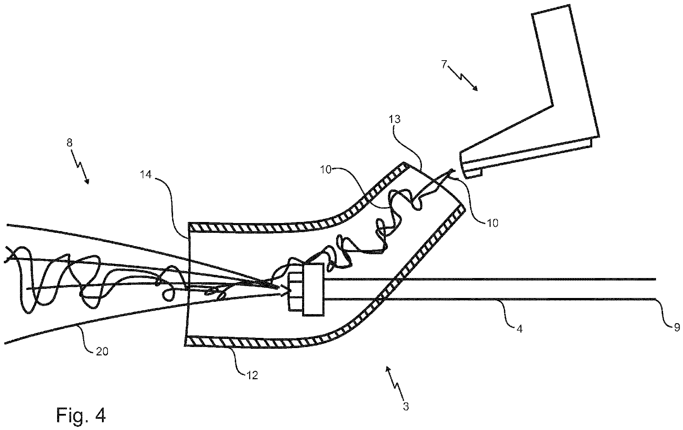

Figure 4 illustrates a further embodiment in which the arthropods 10 are not just falling into the spray jet 8 due to gravity, but are accelerated in the direction of the spray jet 8 through the shielding 12. In this embodiment the shielding 12 is formed tubular and has an opening 13 upstream with respect to the nozzle 3. The shielding 12 and the nozzle 3 are forming a (water) jet pump which sucks the arthropods 10 into the opening 13 and accelerate them in the direction towards the second opening 14 of the shielding, which is located downstream of the nozzle. This acceleration of the arthropods 10 has been found to reduce the speed differences between arthropods 10 and the liquid medium 20 at the point, where the arthropods 10 enter the spray jet 8, and thereby reducing mortality of the arthropods 10.

The shown embodiments all use gravity to emit arthropods 10 through the feeding device 7 from the reservoir 2. However, it is also possible to use other techniques and feeding devices 7. One possibility is an active feeding device 7, by which a predetermined amount of arthropods 10 is transported from the reservoir 2 to the spray jet 8 (or nozzle 3). In this case it is preferred, that during this passage the arthropods 10 are never exposed to the surrounding conditions but are always transported through tubes of the spraying device 1.

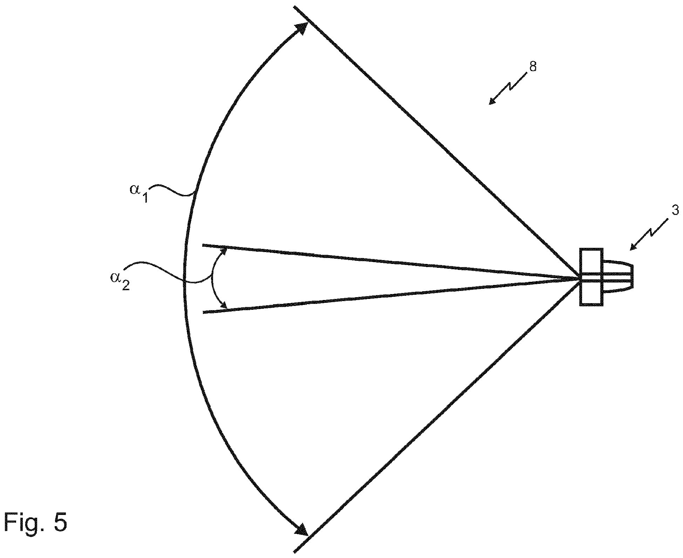

Figures 5 to 7 show several details of a preferred embodiment of the invention. Figure 5 shows a nozzle 3, which allows adjustment of the opening angle $\alpha$. The opening angle $\alpha$ can for example vary in the range between 10° and 180° degrees. A small opening angle $\alpha_2$ can be suitable to direct arthropods 10 by a focused spray jet 8 over a quite long distance to a target area. This could be advantageous if only certain parts of a plant are a suitable habitat for the arthropods 10 to be sprayed. In this case it will be possible to spray arthropods 10 quite precisely only to those parts. In contrast thereto, many applications require a very homogeneous distribution of the arthropods 10. In this case, a wide opening angle $\alpha_1$ would be advantageous, which allows many plants to be treated at the same time. By treating a plurality of plants simultaneously (using a wide opening angle $\alpha_1$) speeds up the process and reduces labor costs.

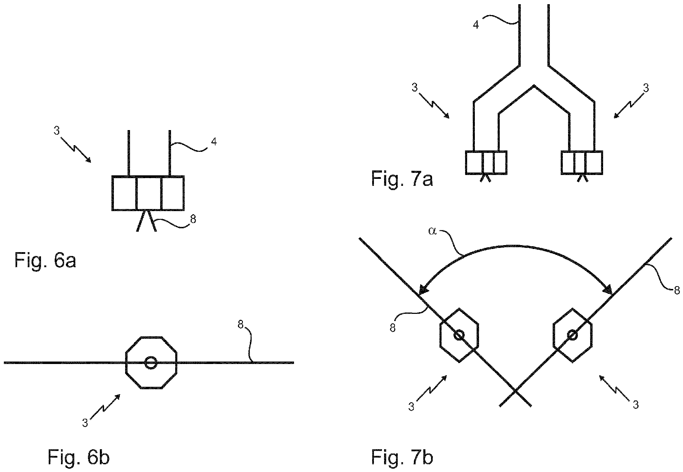

Figures 6a and 6b illustrates a further advantageous embodiment of the present invention. In this embodiment, the spray jet does not have a conical shape (or the shape of a frustum of a cone) but an elliptical or rectangular shape. Even if the spray jet 8 is illustrated as line in figure 6b, it has a finite high. Usually such a spray jet 8 has an elliptical shape with a very small minor axis (defining the height of the spray jet 8). Usually the height of the spray jet 8 is neglectable with respect to its width. Thus, a very flat spray jet 8 like it is shown in figure 6b can be assumed. Such a shape can be advantageous since the amount of liquid medium and/or arthropods 10 can be defined very precisely and is evenly distributed on the crop. One example is the application via a motorized vehicle which passes through the crop. If the arthropods 10 were applied by a conically shaped spray jet 8 (reaching from the top of the plants to the ground), the center region of each plant would be treated longer by the spray jet 8 and thus, more arthropods 10 would be applied. If - in contrast thereto a very flat spray jet 8 (having the same height but a very small width) is used, the treatment period of the center region of each plant will be more or less the same as those of all other parts of the plants and thus, the arthropods 10 will be distributed over each plant very homogenously.

A further preferred embodiment is illustrated in figures 7a and 7b in more detail. This embodiment provides two nozzles 3. These nozzles 3 are spaced apart from each other. They can be directed parallel to each other (like it is illustrated in figure 7a) or inclined about an angle with respect to each other. Using more than only one nozzle 3 can have several advantages. An advantage could be, that at the same time more liquid medium and/or arthropods 10 could be applied to the crop. This would reduce labor costs. A further advantage of more than only one nozzle 3 can be that a very wide opening angle $\alpha$ could be realized. This could be advantageous if the distance between the nozzles and the plants is very small and/or the plants are very high. As illustrated in Figure 7b, individual spray jets 8 could even be inclined with respect to each other.

The size of each hole is preferably very small. In an exemplary embodiment, the diameter of each hole is 0.66 mm and the fluid medium (in this case water) is applied with a pressure of 2 bars at a flow rate of 1.29 liters per minute. The flow speed of the water is in this case 1.57 meters per second.

The spreading device spreads the fluid into the crops, arthropods are added by the dosing unit. The spreading unit of the preferred embodiment is equipped with two nozzles positioned in an angle $\alpha$ which can variant from 180 degrees to 60 degrees. By creating this angle the fluid will create a gutter shape so less material is wasted and the use gets more efficient. Each nozzle is spreading at an exit angle of 80 degrees.

The feeding device of the preferred embodiment feeds the arthropods into the flowing liquid at a speed of release of 30 grams of material per minute and a number of 100 predatory mites per gram, giving a mean number of 50 predators per second. When distributing astigmatid prey mites the system preferably uses the same speed, and a release of a mean number of 30.000 prey mites per gram, so a release of about 15.000 prey mites per second. However, it has to be understood that the astigmatid mites, especially when distributed using a carrier material which offers protection for the mites, can survive higher speeds of spreading compared to predatory mites. Also because the numbers of spread prey mites are supplied on the top of plant preferably *at libitum,* the mortality is not so important and higher mortalities can be accepted without changing the aim of properly feeding the predators at the top of the plants. The skilled person will know which species of arthropods are more sensitive than other to mechanical damages while spreading. When arthropods tolerating high pressures should be sprayed, devices with only one nozzle can be used with the same pressure. In contrast thereto a device with preferably 2 or 3 nozzles is used when spreading (more pressure sensitive) predator mites in order to decrease the mortalities of these mites due to the spreading system.

The first reservoir 2 holds the arthropods for the feeding device 7. The volume is in the range of 0.5 to 5 liters.

The control unit 6 is a fluid regulator, which doses the flow of water depending on the pressure.

Optionally a battery support (not shown) is provided. The battery support holds the power supply for the feeding device 7.

The advantages of the present invention are further illustrated by following trials.

Trial 1

This trial was conducted to evaluate the extent of the distribution of the prey mite *Tyrolichus casei* using the method and device described by the invention compared to a machine and method commercialized in Holland under the name of BioSpreader.

Material and methods

A bag of 5 liters of prey mites, sold under the trade name of Powerfood was used for the trials. This is the product commercialized by Agrobío S.L. which is being introduced into several horticultural and ornamental crops, as an in-crop food to favor the development of the predator populations. The Powerfood bag contains a population of the astigmatid mite *Tyrolichus casei,* packaged with bran and vermiculite as carrier material and/or food for the prey mite, with a density of prey mites of about 10 million mobile stages per liter.

The machine, named "BioSpreader", sold by Pullens BV, was chosen as a standard device. It is currently being used in chrysanthemum crops to spread Powerfood material (live prey mites) into the top of the crop, and is hung on the irrigation system at about 1.4 meters above the crop. One machine is capable of spreading the mites over a width of 1.6 meters, and 4 machines are hung per irrigation system, then covering the current width of a bed, aprox. 6 meters, when using a modern overhead watering device.

To test the distances at which the mites are spread using the Biospreader, a machine was placed at a height of about 140 cm above the ground. A volume of 2 liters of Powerfood was placed in the hoper. Samples of the spread material were collected by placing 3 plates of 20 cm diameter on the ground at 30, 60 and 120 cm distance from the central point above which the machine was hanging (fig. 1). Total amount of mites collected on each plate was estimated under the binocular using the technique of sieving and quartering described by Griffiths et al. Samples of 40 ml from each plate were collected at 30 and 60 cm, but only a few ml of material were collected at 120 cm. To count the mites at 30 and 60 cm, a sieve of 0.5 mm size of gauze was used, and the sieved material was divided 9 consecutive times so that a range of between 150 and 250 individuals were counted under the binocular.

At 120 cm the collected volume was very small, so direct counts of the material from each whole sample were made, using a binocular microscope.

To evaluate the finite distance of the spread following the invention, a trial was performed using the same population of prey mites, from the same bag of Powerfood tested with the BioSpreader. The container of the inventive device was filled with 2 liters of Powerfood, and the device was held on a pole at 1.4 meters above the ground. In front of the device a canvas was placed on the ground and 3 plates (9 cm diameter) were placed on the top of the canvas at distances of 1, 2, 3 and 4 meters, giving a total of 12 plates. The device distributed the material for 3 seconds. The plates were collected and sealed to avoid the escape of mites, then the total amount of mites in each plate was counted under the binocular microscope.

Results

Table 1 shows the number of mites counted per sample at each distance and the mean (±S.E.) number of mites collected at each distance from the Biospreader. Some 48% of the total volume of collected material was obtained at 30 cm distance and 51% at 60 cm, with a mean number of 357.717 and 378.880 mites per sample of 40 ml, respectively. Only a few big pieces of bran were collected at 120 cm, with very few mites (table 1).

Table 1. Number of living mites estimated on each plate located at 30 and 60 cm distance from the BioSpreader, total number of living mites counted in the plates located at 120 cm, and mean number (±S.E.) calculated at each distance.

| Distance (cm) | Replicates | Number of mites | Mean | S.E. |
| --- | --- | --- | --- | --- |
| 30 | 1 | 358,400 | | |
| 30 | 2 | 344,064 | | |
| 30 | 3 | 370,688 | 357,717.3 | 9,422.28 |
| 60 | 1 | 380,928 | | |
| 60 | 2 | 360,448 | | |
| 60 | 3 | 395,264 | 378,880.0 | 12,373.04 |
| 120 | 1 | 25 | | |
| 120 | 2 | 36 | | |
| 120 | 3 | 19 | 26.7 | 6.10 |

Table 2 shows the number of mites counted on plates displaced at 1, 2, 3 and 4 m distances from the nozzle of a device according to the present invention, showing the distribution of prey mites up to 4 meters away.

Table 2. Total number of living mites on plates located at 1, 2, 3 and 4 m distance from the inventive device, and mean (±SE) number of mites calculated at each distance.

| Distance (m) | Replicates | Number of mites | Mean | S.E. |
| --- | --- | --- | --- | --- |
| 1 | 1 | 118 | | |
| 1 | 2 | 87 | | |
| 1 | 3 | 80 | 95.0 | 14.30 |
| 2 | 1 | 95 | | |
| 2 | 2 | 130 | | |
| 2 | 3 | 117 | 114.0 | 12.51 |
| 3 | 1 | 51 | | |
| 3 | 2 | 18 | | |
| 3 | 3 | 32 | 33.7 | 11.71 |
| 4 | 1 | 7 | | |
| 4 | 2 | 2 | | |
| 4 | 3 | 13 | 7.3 | 3.89 |

Compared to the BioSpreader the device of the present invention has shown the ability to distribute the prey mites some 3 meters further.

Trial 2

A trial was performed to evaluate the mortality of the mites after distribution with the invention, and further to evaluate the mortality of prey mites distributed using the method and device of the invention, the number of live and dead mites were counted using the same samples from Trial 1 when the invented device was in operation.

The percentage mortality, after spreading, was calculated according to:

$$(Dead\ mites\ /\ total\ number\ of\ mites)\ x\ 100$$

To calculate the natural mortality of the samples before distribution, 3 samples of 40 ml were collected from the bag of Powerfood. The number of live and dead mites were counted with the same technique of sieving and quartering described by Griffiths. The mortality attributed to the method, after spreading the mites, was estimated as the mortality counted on the plates minus the natural mortality counted in material taken from the bag when it was first opened.

Results

Table 3 shows the number of dead and live mites after spreading following the inventive method and device. The sampling of the material before spreading showed a mean mortality of 4.87%. The mortality due to the spreading process (corrected mortality) was then calculated by subtracting 4.87% from the calculated mortality in the sample.
The results show that the mean mortalities at different distances from the nozzle were quite similar; below a variation of 10 % in all cases.

Table 3. Total number of living and dead mites, mortality and corrected mortality on plates located at 1, 2, 3 and 4 m distance from the inventive device, and mean ($\pm$SE) corrected mortality of mites calculated at each distance.

| Distance (m) | Replicates | Living mites | Dead mites | % mortality | Corrected mortality | Mean | SE |
| --- | --- | --- | --- | --- | --- | --- | --- |
| 1 | 1 | 118 | 14 | 11.9 | 7.0 | | |
| 1 | 2 | 87 | 9 | 10.3 | 5.5 | | |
| 1 | 3 | 80 | 9 | 11.3 | 6.4 | 6.3 | 0.54 |
| 2 | 1 | 95 | 12 | 12.6 | 7.8 | | |
| 2 | 2 | 130 | 14 | 10.8 | 5.9 | | |
| 2 | 3 | 117 | 19 | 16.2 | 11.4 | 8.3 | 1.97 |
| 3 | 1 | 51 | 7 | 13.7 | 8.9 | | |
| 3 | 2 | 18 | 3 | 16.7 | 11.8 | | |
| 3 | 3 | 32 | 4 | 12.5 | 7.6 | 9.4 | 1.51 |
| 4 | 1 | 7 | 1 | 14.3 | 9.4 | | |
| 4 | 2 | 2 | 0 | 0.0 | 0.0 | | |
| 4 | 3 | 13 | 2 | 15.4 | 10.5 | 6.6 | 6.07 |

Third trial

A trial was performed on a chrysanthemum crop to evaluate the establishment of predatory mites in the crop using the method and device described in the patent. The aim was to distribute populations of the predatory mite *Transeius montdorensis* in a crop at the seedling stage to ensure an early establishment of the predator and so improve the degree of control of the main pest, the thrips.
This is a long term, unresolved problem because the wholesale buyers of Chrysanthemums will only accept a very low threshold of thrip damage since it is directly related to scarring of the petals. The problem is exacerbated because fewer and fewer active ingredients are being authorized for inclusion in pesticides. Thus, continued use of those available quickly make registered chemicals less and less effective due to pesticide resistance.
Two years ago, the company Agrobío developed a system based on introduction of predatory populations and additional releases of populations of prey mites as an in-crop food. This introduction of the complementary food considered improved the establishment of the predators. A program was set up which is based on 4 weekly releases of both the predatory mite *Transeius montdorensis* and pure populations of its prey mite, followed by a further two weekly introductions of the pure prey mite.
A machine called BioSpreader is used to spread both populations of mites. However, the machine is not designed to spread mites on the seedlings whilst they are still in the nursery prior to planting. For this reason, the mites are released about two weeks after transplanting the crop.
The present invention solves this problem in that now it will be possible to apply the beneficial mites whilst the small plants are still in the nursery. In the present trial, the method and device described in the invention was used to spread the mites five days before transplanting and four days after transplanting (week 1). Afterwards, the standard series of releases were applied between week 2 and 4.

The establishment of these predators was then compared to a control treatment where prey and predators were released on a weekly base between week 2 and 4 using the BioSpreader. All populations of *T. montdorensis* and prey mites used in the trials were supplied by Agrobio, corresponding to the commercial products MontControl (paper bags with 5 liters contents) and Powerfood (paper bags also with 5 liters). The trial was performed in one commercial greenhouse situated in the Dutch province of Gelderland, with a surface area of 6,000 $m^2$ of plants of the variety *chrysanthemum raisa.* Following the common practice of the producers, the greenhouse was divided in plots of approx. 1,000 $m^2$, with a crop duration of 10 weeks.

Releases according to the invention

On nursery plants

Releases of both predators and preys were completed on all the plants in the nursery five days before transplanting. Using the invention, the material contained in two MontControl and two Powerfood units was spread over the top of every 180,000 plants (to be transplanted on approx. 3,000 $m^2$), giving an estimated ratio of 1.39 predators released per plant, together with their prey mites *ad libitum* (more than 140 preys per plant).

Releases after transplanting

After removal from the release nursery, additional releases were performed on the crop (3,000 $m^2$) of one Powerfood and one MontControl per 1,000 $m^2$ on weeks 1 and 2, followed by two Powerfood and two MontControl per 1,000 $m^2$ on week 3, and then two Powerfood and three MontControl per 1,000 $m^2$ on week 4.

Releases using BioSpreader

Commercial standard programmes of releases were performed using the BioSpreader on 3,000 $m^2$. Ratios and timing of the releases per 1,000 $m^2$ were: 1-2-3 Montcontrol bags on weeks 2, 3, and 4, respectively, and 1-2-2 Powerfood bags on weeks 2, 3, and 4, respectively. This means a total release estimated of about 12 *T. montdorensis* per plant and 25 ml of food per $m^2$.

Sampling

To evaluate possible losses of mites caused during handling and/or due to the water stress (no irrigation) commonly performed after transplanting, sampling was performed just before transplanting (week 0) and 4-5 days after transplanting (week 1), in the treatment of releases using the invention. A total of 10 randomly collected plants were sampled. The following weeks 2 to 4, 6 plants per treatment were sampled weekly to evaluate the development of the predator and pest populations.

Data analysis

All sampled plants were packaged in plastic bags, cut into small pieces and examined using a binocular microscope in the laboratory. Total number of eggs and mobile stages of predatory mites, mobile stages of prey mites, and larvae and adults of thrips per plant were counted. Number of leaves per plant were also counted. Means ($\pm$S.E.) of predators and thrips per each weekly treatment were estimated.

Results

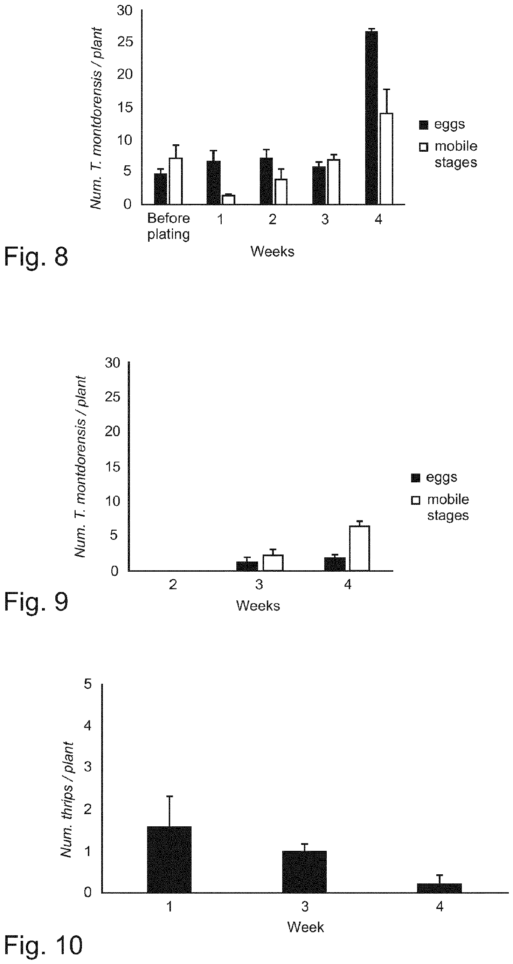

The mean number ($\pm$S.E.) of mobile stages and eggs of *T. montdorensis* on each sampling date in the plot where the mites were spread using the invention and in the plot where BioSpreader was used are represented in figures 1 and 2, respectively. In the plot where the mites were distributed over seedlings, whilst using the invention, a considerable number of individuals were counted in these small plants before and after transplanting, at which time the plants have only 1-2 leaves. These very young plants were found to have between 4 and 6 eggs of *T. montdorensis* per plant, showing a good early establishment of the predators. Importantly, there were no thrips on these plants. But, in the treatment using the BioSpreader (Figure 10) a week after transplanting (week 1) the mean number of thrips had risen to 1.6.

On weeks 1 and 4 an accumulative number of 8.3 and 26 mobile stages of *T. montdorensis* and an accumulative number of 3.1 and 45.9 eggs of the predator occurs when using BioSpreader or the invention, respectively. This means a 3 times higher number of mobile stages and almost 15 times higher number of eggs when using the invention compared to the BioSpreader.

Although the numbers of thrips are reduced when the predator populations increase in the treatment with BioSpreader, on week 4 (fig. 10), the control is not enough to guarantee that damages do not occur during the first weeks, when thrips are already present and predators are not yet established. In contrast, the invented method and device promotes the establishment of a higher number of predators per plant from the early stages and this clearly explains why zero thrip populations were counted in the plants treated according to the present invention.

The high number of predators established when using the method according to the present invention shows, that this system can be used to spread both predator and pure prey mite populations. The invention favours the development of higher populations compared to other systems already commercially available in the market. This may be explained both because it can be used from early stages and because it promotes the survival of the prey mite populations, leading to the faster establishment of the predators. This may be partially explained because the extra humidity is provided whilst distributing the mites.

It will be understood that the embodiments explained above are merely a first embodiment of the method and/or system of the invention. In this respect, the disclosure of the invention is not limited to these embodiments.

All the features disclosed in the application documents are claimed as being essential to the invention in so far as they are individually or in combination novel over the prior art.

**Claims**

1. A method for distributing arthropods in a crop by
providing a flow of a liquid medium through a tube in a direction towards a first end of the tube,
providing arthropods in a first container,
adding arthropods to the liquid medium,
allowing the liquid medium to exit the tube at a first end and
spraying the mixture of liquid medium and arthropods to a target.

2. Method according to claim 1,
**characterized in that**
the liquid medium is provided from a reservoir in which it is maintained at a pressure between 10 kPa and 1000 kPa, preferably between 100 and 500 kPa, more preferably between 200 and 400 kPa above the atmospheric pressure.

3. Method according to claim 1 or 2,
**characterized by**
adjusting the length and/or width of the spray jet by amending features selected from the group comprising the pressure of the liquid medium in the reservoir, the flow velocity of the liquid medium, the diameter of a section of a tube connecting a reservoir for the liquid medium and the nozzle, the density of the liquid medium, the viscosity of the liquid medium, the cross sectional area of the nozzle and other characteristics of the nozzle.

4. Method according to any of the preceding claims,
**characterized by**
providing a food source for the arthropods to and/or together with the liquid medium.

5. Method according to any of the preceding claims,
**characterized by**
providing the arthropods together with a carrier to the liquid medium, wherein the carrier is preferably selected from a group comprising vermiculite, particles of perlite, saw dust, wood dust, bran, buckwheat, shells of seeds and mixtures thereof.

6. Method according to any of the preceding claims,
**characterized by**
spraying the mixture of dispersed liquid and arthropods over a maximum horizontal distance of at least 150 cm, preferably of at least 200 cm, more preferably of at least 300 cm and especially preferably of at least 500 cm.

7. Method according to any of the preceding claims,
**characterized by**
selecting the arthropods species from one or more of insects and mites, preferably from the order Mesostigmata, more preferably from the family Phytoseiidae, most preferably from a group comprising *Amblyseius swirskii, Amblyseius andersoni, Amblyseius largoensis, Euseius stipulatus, Euseius scutalis, Euseius gallicus, Euseius ovalis, Euseius finlandicus, Neoseiulus californicus, Neoseiulus cucumeris, Neoseiulus fallacis, Neoseiulus longispinosus, Amblydromalus limonicus, Transeius montdorensis, Phytoseiulus persimilis* and *Phytoseiulus longipes,* or

from the order Astigmata, more preferably selected from a group comprising the genera *Acarus, Carpoglyphus, Tyrolichus, Thyreophagus, Suidasia, Lepidoglyphus* and *Aleuroglyphus,* most preferably from a group comprising *Acarus siro, Carpoglyphus lactis, Tyrolichus casei, Thyreophagus entomophagus, Aleuroglyphus ovatus, Lepidoglyphus destructor* and *Suidasia medanendis.*

8. A device for spraying arthropods from a first reservoir to a target, wherein the device comprises a second reservoir for a liquid medium which is in fluid connection to a first end of a tube from which the liquid medium exits the tube, **characterized in that** the first reservoir comprises a feeding device by which arthropods can be fed to the liquid medium.

9. A device according to claim 8,
**characterized in that**
the device comprises adjustment means for allowing and/or preventing flow of the liquid medium through the first end of a tube which is preferably a nozzle and/or adapting parameters of the spray jet selected from the group comprising the lengths, width, height, opening angle, diameter, form, velocity, flow rate of the spray jet.

10. A device according to claims 8 or 9,
**characterized in that**
the second reservoir for the liquid media is portable, preferably portable together with the nozzle, most preferably both located on a vehicle.

11. A device according to at least one of claims 8 to 10,
**characterized in that**
the first reservoir comprises a dosing device by which the number and/or weight of emitted arthropods per minute and/or volume of sprayed liquid medium can be adjusted.

12. A device according to at least one of claims 8 to 11,
**characterized in that**
the arthropods located in the first reservoir are one or more species of insects and mites,
preferably selected
from the order Mesostigmata, more preferably selected from the family Phytoseiidae, most preferably from a group comprising *Amblyseius swirskii, Amblyseius andersoni, Amblyseius largoensis, Euseius stipulatus, Euseius scutalis, Euseius gallicus, Euseius ovalis, Euseius finlandicus, Neoseiulus californicus, Neoseiulus cucumeris, Neoseiulus fallacis, Neoseiulus longispinosus, Amblydromalus limonicus, Transeius montdorensis, Phytoseiulus persimilis* and *Phytoseiulus longipes,* or
from the order Astigmata, more preferably selected from a group comprising the genera *Acarus, Carpoglyphus, Tyrolichus, Thyreophagus, Suidasia, Lepidoglyphus* and *Aleuroglyphus,* most preferably from a group comprising *Acarus siro, Carpoglyphus lactis, Tyrolichus casei, Thyreophagus entomophagus, Aleuroglyphus ovatus, Lepidoglyphus destructor* and *Suidasia medanendis;*
wherein preferably the first reservoir further comprises a carrier which is selected from a group comprising vermiculite, particles of perlite, saw dust, wood dust, bran, buckwheat, shells of seeds and mixtures thereof.

13. A device according to at least one of claims 8 to 12,
**characterized in that**
the device comprises means for adding a food source for the arthropods to the liquid media, wherein the food source is preferably selected from a group comprising pollen, sugar, amino acids, vitamins, nutrients derived from insect tissue, eggs, preferably mite eggs, moths, cysts and/or mites, preferably dead mites.

14. A device according to at least one of claims 8 to 13,
**characterized in that**
the feeding device, by which arthropods can be delivered to the spray jet, is located 0.01 - 200 cm, preferably 1 - 50 cm, more preferably 2 - 10 cm downstream of the nozzle.

15. A method for protecting a crop by providing at least one beneficial arthropod in the crop and

providing a food source for the beneficial arthropod species according to the method of one of claims 1 to 7 and/or by means of a device according to one of claims 8 to 14.

**Amended claims in accordance with Rule 137(2) EPC.**

1. A method for distributing arthropods in a crop by
providing a flow of a liquid medium through a tube in a direction towards a first end of the tube,
providing arthropods in a first container,
adding arthropods to the flow of the liquid medium within a distance between less than 20 cm upstream the first end of the tube and less than 200 cm downstream of the first end of the tube,
allowing the liquid medium to exit the tube at a first end and
spraying the mixture of liquid medium and arthropods to a target.

2. Method according to claim 1,
**characterized in that**
the liquid medium is provided from a reservoir in which it is maintained at a pressure between 10 kPa and 1000 kPa, preferably between 100 and 500 kPa, more preferably between 200 and 400 kPa above the atmospheric pressure.

3. Method according to claim 1 or 2,
**characterized by**
adjusting the length and/or width of the spray jet by amending features selected from the group comprising the pressure of the liquid medium in the reservoir, the flow velocity of the liquid medium, the diameter of a section of a tube connecting a reservoir for the liquid medium and the nozzle, the density of the liquid medium, the viscosity of the liquid medium, the cross sectional area of the nozzle and other characteristics of the nozzle.

4. Method according to any of the preceding claims,
**characterized by**
providing a food source for the arthropods to and/or together with the liquid medium.

5. Method according to any of the preceding claims,
**characterized by**
providing the arthropods together with a carrier to the liquid medium, wherein the carrier is preferably selected from a group comprising vermiculite, particles of perlite, saw dust, wood dust, bran, buckwheat, shells of seeds and mixtures thereof.

6. Method according to any of the preceding claims,
**characterized by**
spraying the mixture of dispersed liquid and arthropods over a maximum horizontal distance of at least 150 cm, preferably of at least 200 cm, more preferably of at least 300 cm and especially preferably of at least 500 cm.

7. Method according to any of the preceding claims,
**characterized by**
selecting the arthropods species from one or more of insects and mites, preferably from the order Mesostigmata, more preferably from the family Phytoseiidae, most preferably from a group comprising *Amblyseius swirskii, Amblyseius andersoni, Amblyseius largoensis, Euseius stipulatus, Euseius scutalis, Euseius gallicus, Euseius ovalis, Euseius finlandicus, Neoseiulus californicus, Neoseiulus cucumeris, Neoseiulus fallacis, Neoseiulus longispinosus, Amblydromalus limonicus, Transeius montdorensis, Phytoseiulus persimilis* and *Phytoseiulus longipes,* or
from the order Astigmata, more preferably selected from a group comprising the genera *Acarus, Carpoglyphus, Tyrolichus, Thyreophagus, Suidasia, Lepidoglyphus* and *Aleuroglyphus,* most preferably from a group comprising *Acarus siro, Carpoglyphus lactis, Tyrolichus casei, Thyreophagus entomophagus, Aleuroglyphus ovatus, Lepidoglyphus destructor* and *Suidasia medanendis.*

8. A device for spraying arthropods from a first reservoir to a target, wherein the device comprises a second reservoir for a liquid medium which is in fluid connection to a first end of a tube from which the liquid medium exits the tube, **characterized in that** the first reservoir comprises a feeding device by which arthropods can be fed to the liquid medium within a distance between less than 20 cm upstream the first end of the tube and less than 200 cm downstream of the first end of the tube, wherein the first end of the tube is an adjustable nozzle.

9. A device according to claim 8,
   **characterized in that**
   the device comprises adjustment means for allowing and/or preventing flow of the liquid medium through the first end of a tube which is preferably a nozzle and/or adapting parameters of the spray jet selected from the group comprising the lengths, width, height, opening angle, diameter, form, velocity, flow rate of the spray jet.

10. A device according to claims 8 or 9,
    **characterized in that**
    the second reservoir for the liquid media is portable, preferably portable together with the nozzle, most preferably both located on a vehicle.

11. A device according to at least one of claims 8 to 10,
    **characterized in that**
    the first reservoir comprises a dosing device by which the number and/or weight of emitted arthropods per minute and/or volume of sprayed liquid medium can be adjusted.

12. A device according to at least one of claims 8 to 11,
    **characterized in that**
    the arthropods located in the first reservoir are one or more species of insects and mites,
    preferably selected
    from the order Mesostigmata, more preferably selected from the family Phytoseiidae, most preferably from a group comprising *Amblyseius swirskii, Amblyseius andersoni, Amblyseius largoensis, Euseius stipulatus, Euseius scutalis, Euseius gallicus, Euseius ovalis, Euseius finlandicus, Neoseiulus californicus, Neoseiulus cucumeris, Neoseiulus fallacis, Neoseiulus longispinosus, Amblydromalus limonicus, Transeius montdorensis, Phytoseiulus persimilis* and *Phytoseiulus longipes,* or
    from the order Astigmata, more preferably selected from a group comprising the genera *Acarus, Carpoglyphus, Tyrolichus, Thyreophagus, Suidasia, Lepidoglyphus* and *Aleuroglyphus,* most preferably from a group comprising *Acarus siro, Carpoglyphus lactis, Tyrolichus casei, Thyreophagus entomophagus, Aleuroglyphus ovatus, Lepidoglyphus destructor* and *Suidasia medanendis;*
    wherein preferably the first reservoir further comprises a carrier which is selected from a group comprising vermiculite, particles of perlite, saw dust, wood dust, bran, buckwheat, shells of seeds and mixtures thereof.

13. A device according to at least one of claims 8 to 12,
    **characterized in that**
    the device comprises means for adding a food source for the arthropods to the liquid media, wherein the food source is preferably selected from a group comprising pollen, sugar, amino acids, vitamins, nutrients derived from insect tissue, eggs, preferably mite eggs, moths, cysts and/or mites, preferably dead mites.

14. A device according to at least one of claims 8 to 13,
    **characterized in that**
    the feeding device, by which arthropods can be delivered to the spray jet, is located 0.01 - 200 cm, preferably 1 - 50 cm, more preferably 2 - 10 cm downstream of the nozzle.

15. A method for protecting a crop by providing at least one beneficial arthropod in the crop and
    providing a food source for the beneficial arthropod species according to the method of one of claims 1 to 7 and/or by means of a device according to one of claims 8 to 14.

Fig. 1

Fig. 2

Fig. 3

Fig. 4

Fig. 5

Fig. 6a

Fig. 6b

Fig. 7a

Fig. 7b

Fig. 8

Fig. 9

Fig. 10

Europäisches
Patentamt

European
Patent Office

Office européen
des brevets

**EUROPEAN SEARCH REPORT**

Application Number

EP 18 15 6799

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 97/07898 A1 (US AGRICULTURE [US]) 6 March 1997 (1997-03-06) | 1-3,5,7 | INV. A01M7/00 |
| Y | * page 1, lines 3-9 * <br> * page 2, line 38 - page 3, line 29 * <br> * page 4, lines 3-11 * <br> * page 5, lines 19-25 * <br> * page 8, lines 10-26 * <br> * page 9, lines 10-15 * <br> * page 9, line 36 - page 10, line 1 * <br> * figures 1,3 * | 4,6,8-15 | A01M9/00 |
| Y,D | WO 2007/136246 A1 (KOPPERT BV [NL]; TETTEROO ADRIANUS NICOLAAS MAR [NL]; VEENMAN AREND [N) 29 November 2007 (2007-11-29) <br> * page 1, lines 3-9 * <br> * page 2, line 16 - page 4, line 23 * <br> * page 6, line 7 - page 7, line 9 * <br> * page 9, lines 9-17 * <br> * page 10, lines 3-23 * <br> * page 11, lines 18-21 * <br> * page 12, line 1 - page 13, line 14 * <br> * page 15, lines 1-12 * <br> * page 16, line 22 - page 17, line 10 * <br> * figure 1 * | 6,8-12, 14,15 | |
| Y | WO 2007/075081 A1 (KOPPERT BV [NL]; BOLCKMANS KAREL JOZEF FLORENT [BE]; VAN HOUTEN YVONNE) 5 July 2007 (2007-07-05) <br> * page 1, lines 6-8,20-22 * <br> * page 12, lines 16-26 * <br> * page 17, lines 27-31 * <br> * page 23, lines 2-14 * | 4,13 | TECHNICAL FIELDS SEARCHED (IPC) <br><br> A01M |

-/--

The present search report has been drawn up for all claims

| Place of search | Date of completion of the search | Examiner |
|---|---|---|
| Munich | 22 August 2018 | Schlichting, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 18 15 6799

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/103295 A1 (KOPPERT BV [NL]; BOLCKMANS KAREL JOZEF FLORENT [BE]; VAN HOUTEN YVONNE) 11 July 2013 (2013-07-11)<br>* page 2, line 31 - page 4, line 14 *<br>* page 4, lines 28-29 *<br>* page 6, lines 21-28 *<br>* page 11, line 26 - page 12, line 14 *<br>----- | 5,7,12 | |
| A | WO 2010/004556 A2 (AGRICULTURAL RES ORG [IL]; GAN-MOR SHMUEL [IL]; PALEVSKY ERIC [IL]; RO) 14 January 2010 (2010-01-14)<br>* page 4, line 25 - page 5, line 26 *<br>* figure 1 *<br>----- | 4,13 | |

TECHNICAL FIELDS
SEARCHED (IPC)

The present search report has been drawn up for all claims

| Place of search | Date of completion of the search | Examiner |
|---|---|---|
| Munich | 22 August 2018 | Schlichting, N |

EPO FORM 1503 03.82 (P04C01)

**ANNEX TO THE EUROPEAN SEARCH REPORT
ON EUROPEAN PATENT APPLICATION NO.**                    EP 18 15 6799

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2018

| Patent document cited in search report | | Publication date | Patent family member(s) | | Publication date |
|---|---|---|---|---|---|
| WO 9707898 | A1 | 06-03-1997 | US | 5785245 A | 28-07-1998 |
| | | | WO | 9707898 A1 | 06-03-1997 |
| WO 2007136246 | A1 | 29-11-2007 | EC | SP088915 A | 30-12-2008 |
| | | | EP | 2018100 A1 | 28-01-2009 |
| | | | NL | 1032099 C2 | 23-11-2007 |
| | | | WO | 2007136246 A1 | 29-11-2007 |
| WO 2007075081 | A1 | 05-07-2007 | AP | 2977 A | 30-09-2014 |
| | | | AT | 476099 T | 15-08-2010 |
| | | | AU | 2005339589 A1 | 05-07-2007 |
| | | | BR | PI0520809 A2 | 23-06-2009 |
| | | | CA | 2635546 A1 | 05-07-2007 |
| | | | CN | 101374409 A | 25-02-2009 |
| | | | DK | 1965634 T3 | 15-11-2010 |
| | | | EA | 200870126 A1 | 27-02-2009 |
| | | | EC | SP088586 A | 30-07-2008 |
| | | | EP | 1965634 A1 | 10-09-2008 |
| | | | ES | 2349941 T3 | 13-01-2011 |
| | | | IL | 192463 A | 30-08-2012 |
| | | | JP | 5393155 B2 | 22-01-2014 |
| | | | JP | 2009522256 A | 11-06-2009 |
| | | | KR | 20080085063 A | 22-09-2008 |
| | | | KR | 20140135988 A | 27-11-2014 |
| | | | MX | 277715 B | 30-07-2010 |
| | | | NZ | 569350 A | 28-05-2010 |
| | | | PT | 1965634 E | 10-11-2010 |
| | | | TN | SN08281 A1 | 30-10-2009 |
| | | | US | 2009205057 A1 | 13-08-2009 |
| | | | WO | 2007075081 A1 | 05-07-2007 |
| WO 2013103295 | A1 | 11-07-2013 | BR | 112014016772 A2 | 13-06-2017 |
| | | | CA | 2860644 A1 | 11-07-2013 |
| | | | DK | 2800467 T3 | 03-10-2016 |
| | | | EP | 2800467 A1 | 12-11-2014 |
| | | | EP | 3165087 A1 | 10-05-2017 |
| | | | ES | 2587777 T3 | 26-10-2016 |
| | | | HU | E030450 T2 | 29-05-2017 |
| | | | JP | 2015508289 A | 19-03-2015 |
| | | | JP | 2018019713 A | 08-02-2018 |
| | | | KR | 20140111000 A | 17-09-2014 |
| | | | MA | 35881 B1 | 01-12-2014 |
| | | | PL | 2800467 T3 | 31-01-2017 |
| | | | PT | 2800467 T | 12-10-2016 |
| | | | RU | 2014131953 A | 27-02-2016 |
| | | | UA | 116880 C2 | 25-05-2018 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 15 6799

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2018

| Patent document cited in search report | Publication date | Patent family member(s) | | Publication date |
|---|---|---|---|---|
| | | US | 2015000600 A1 | 01-01-2015 |
| | | WO | 2013103295 A1 | 11-07-2013 |
| WO 2010004556 A2 | 14-01-2010 | EP | 2309844 A2 | 20-04-2011 |
| | | US | 2011162266 A1 | 07-07-2011 |
| | | WO | 2010004556 A2 | 14-01-2010 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007136246 A